# EUROPEAN PATENT SPECIFICATION

(11) **EP 4 042 155 B1**
(45) Date of publication and mention of the grant of the patent: **04.10.2023**
(21) Application number: 20789918.8
(22) Date of filing: 07.10.2020
(51) Int. Cl.: G01N 33/50, G01N 33/543, G01N 33/68

(54) **XBP1 ISOFORM MULTIPLEX ASSAY**
XBP1 ISOFORM MULTIPLEX ASSAY
DOSAGE MULTIPLEX DE L'ISOFORME XBP1

(30) Priority: 08.10.2019 GB 201914517
(43) Date of publication of application: 17.08.2022
(73) Proprietor: Randox Laboratories Ltd., Crumlin Antrim BT29 4QY (GB); Randox Teoranta, Dungloe F94 TV06 (IE)
(72) Inventor: MCCONNELL, Ivan, Crumlin Antrim BT29 4QY (GB); FITZGERALD, Peter, Crumlin Antrim BT29 4QY (GB); RICHARDSON, Ciaran, Dungloe, F94TV06 (IE); MARTIN, Kenneth, Dungloe, F94TV06 (IE)
(74) Representative: Gill Jennings & Every LLP
(86) International application number: PCT/EP2020/078174
(87) International publication number: WO 2021/069524

(56) References cited:
- WO-A2-2005/034737
- GUO F ET AL: "XBP1U inhibits the XBP1S-mediated upregulation of the iNOS gene expression in mammalian ER stress response", CELLULAR SIGNALLING, ELSEVIER SCIENCE LTD, GB, vol. 22, no. 12, 1 December 2010 (2010-12-01), pages 1818-1828, XP027279206, ISSN: 0898-6568 [retrieved on 2010-07-15]

## Description

### BACKGROUND OF THE INVENTION

The unfolded protein response (UPR) is a set of eukaryotic pathways activated by endoplasmic reticulum (ER) stress, defined by an accumulation of unfolded or misfolded proteins in the ER. Canonically, detection of protein misfolding is dependent upon three ER-transmembrane receptors: inositol requiring enzyme 1 (IRE1), protein kinase RNALactivated (PKR)Llike ER kinase (PERK) and activating transcription factor 6 (ATF6). The most studied and characterised of these ER stress sensors is IRE1. Activation of the ubiquitous IRE1α isoform is dependent upon its autophosphorylation and subsequent oligomerisation which results in activation of its cytosolic RNase domain. Signal transduction is possible through both kinase and RNase activity. The most widely studied result of IRE1α activation is the RNase domain mediated, unconventional splicing of XBP1 pre-mRNA resulting in the excision of 26 nucleotides and a frameshift in its open reading frame.

Translation of the conventionally spliced mRNA, XBP1u, results in XBP1u, which has few known functions and is rapidly degraded in vitro. The most characterised action of XBP1u is as a negative regulator of XBP1s, though it has also been linked to regulation of the p53/p21 tumour suppression pathway. In contrast, translation of XBP1s mRNA (the result of unconventional IRE1α mediated XBP1 splicing) produces a potent transcription factor, XBP1s, along with other UPR regulated transcription factors, starts a transcriptional programme aimed at reducing protein load through increased expression of the ER's protein folding or protein degradation machinery. Increased splicing of XBP1 has been associated with disease progression, therapy resistance and as a druggable target in a range of diseases.

The UPR is activated as a key pro-survival mechanism in many solid tumours in response to hypoxic and nutrient deprived conditions. Constitutive activation of IRE1α is proposed to confer cancer cells a selective advantage over neighbouring healthy and non-UPR activated cancer cells, with recent studies demonstrating upregulated XBP1 splicing in breast, pancreatic and ovarian cancer. XBP1s upregulation in immune cells also contributes to immune evasion within the tumour microenvironment. Several conventional therapies routinely used in cancer treatment have also been shown to induce IRE1α RNase activity, either providing a pro-survival resistance or enhancing apoptotic effects. Small molecule targeting of IRE1α RNase activity is being investigated as an adjuvant therapy in several cancers. Elevated levels of XBP1s have also been correlated with a protective effect in neurodegenerative diseases associated with protein misfolding including Alzheimer's, Parkinson's and Huntington's diseases. The consequences of IRE1α activation are highly context dependent, with links to various molecular pathways including autophagy, apoptosis and prion resistance.

As therapies targeting the UPR enter clinical trials and evidence for the use of XBP1s as a pathologically relevant biomarker grows, effective means of monitoring XBP1 splicing and expression of the XBP1 isoforms has become a clinical need. None of the methods currently employed for XBP1s or XBP1u detection are suitable for routine use in a clinical laboratory. RT-PCR and RT-qPCR are often used to assess XBP1 splicing, using primers flanking the spliced intron sequences where variant specificity is required. Factors such as extended sample preparation, potential for contamination and requirements for standardisation and normalisation of results make RT-qPCR unsuitable for medium-high throughput in non-sterile clinical laboratories. At the protein level, standard assessment of XBP1s is performed individually by immunoblotting. Medium-high throughput is not practical with this time-consuming and technically laborious method. Western blots are also largely unsuitable for quantification or inter-blot comparison with variation due to detection mechanism, reagents and analysis methods.

### SUMMARY OF THE INVENTION

The invention is defined in the appended claims.

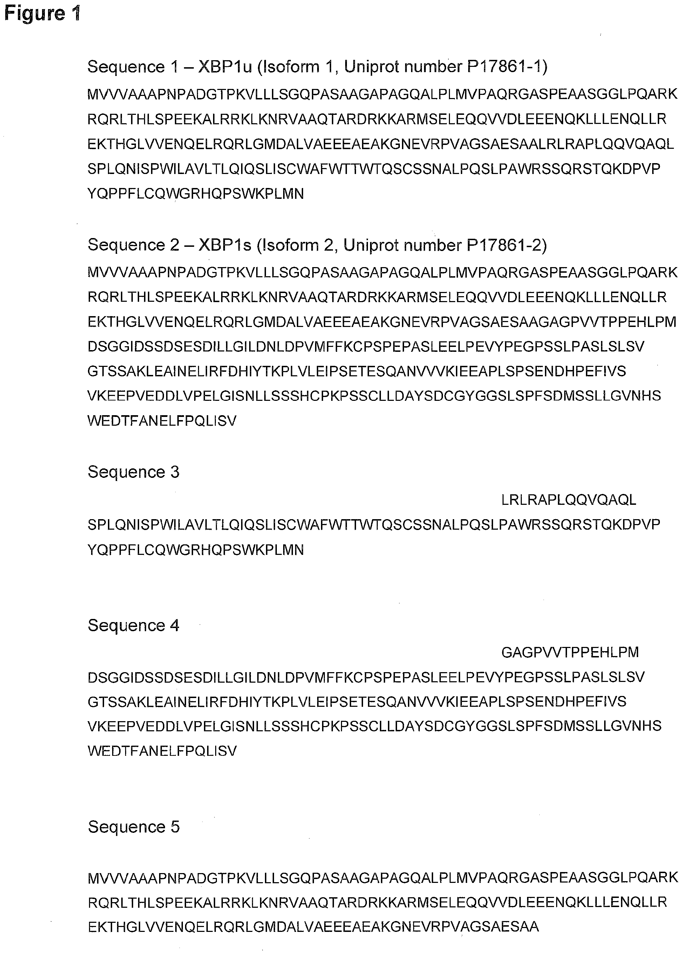

XBP1 exists in two protein isoforms, XBP1u and XBPs, but it is the latter that has been of major clinical interest and subject to targeted assay development for its detection. Both isoforms of XBP1 are becoming implicated in pathogenic or disease progression mechanisms and there is a need for a reliable, simplistic, rapid and clinically applicable method for the detection and determination of both isoforms. An assay system is described for the first time using the principles of a sandwich multianalyte array to simultaneously capture the two XBP1 isoforms utilising their different C-termini, and detecting the captured protein using a pan-detector moiety targeted to their common N-terminus and its application to simultaneously detect the two XBP1 isoforms in models of breast cancer, non-adherent cells and inflammation. In a first aspect, the invention relates to a method of detecting or determining XBP1u and XBP1s in an *in vitro* cell line or an *in vitro* (ex *vivo*) biological sample using a solid-state device which is preferably a biochip, microtitre plate, beads or a plastic slide supporting two antibodies, one antibody specific to XBP1u the second antibody specific to XBP1s, and adding at least one further detector antibody, and detecting or determining the presence or amount of each of XBP1u and XBP1s; preferably, the antibody specific to XBP1u binds to an epitope incorporated within XBP1u of sequence 1 and the antibody specific to XBP1s binds to an epitope incorporated within XBP1s of sequence 2 (sequences shown in Figure 1); preferably the at least one further detector antibody is specific to the same epitope present in both XBP1s and XBP1u.

A further aspect the invention describes a method that enables the simultaneous exposure of the two isoform-specific XBP1 antibodies to an added cell line or biological sample and the simultaneous detection or quantification of the two bound or captured XBP1 protein isoforms.

A further aspect of the invention introduces a method of detecting or determining XBP1u and XBP1s in an *in vitro* cell line or an *in vitro* biological sample using a solid-state device and from which a ratio of the XBP1s and XBP1u isoforms is derived.

In a further aspect, the invention relates to a solid-state device supporting two antibodies, one antibody specific to XBP1u the second antibody specific to XBP1s; the antibody specific to XBP1u binding to an epitope incorporated within XBP1u of sequence 1 the antibody specific to XBP1s binding to an epitope incorporated within XBP1s of sequence 2.

The invention further describes, for the first time, reliable, rapid and simplistic methods of ascertaining the status of the unfolded protein response by way of the detection or determination of both XBP1s and XBP1u protein isoforms, improving upon less reliable XBP1 transcript measurements.

A further aspect of the invention relates to a kit comprising the aforementioned solid-state device.

### Figures

FIGURE 1 Sequences of XBP1u and XBP1s and antibody epitopic binding regions

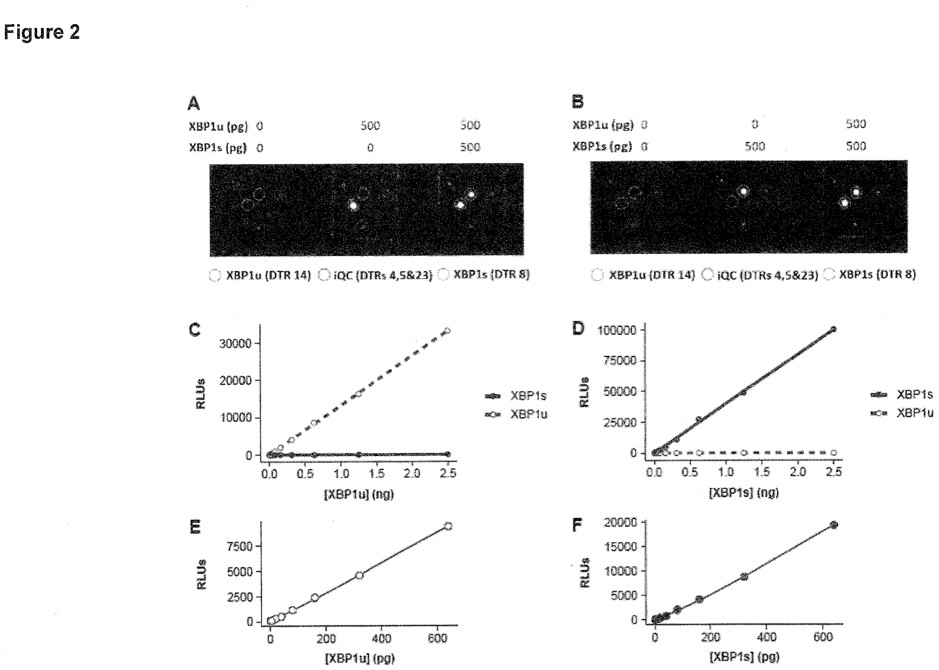

FIGURE 2 XBP1 biochip specificity and signal-antigen relationship upon XBP1s and XBP1u spiking and induction. 500 pg XBP1s and/or XBP1u were spiked alone or in combination into calibrator base material before application to XBP1 biochip with the DTRs indicated, including for internal quality control (iQC) (A, B). XBP1s and XBP1u were individually spiked into calibrator base material at 2.5 ng per sample and serially diluted before application to XBP1 biochip (C, D). XBP1 biochip multiplexed calibration curves from 0-650 pg per sample (E, F).

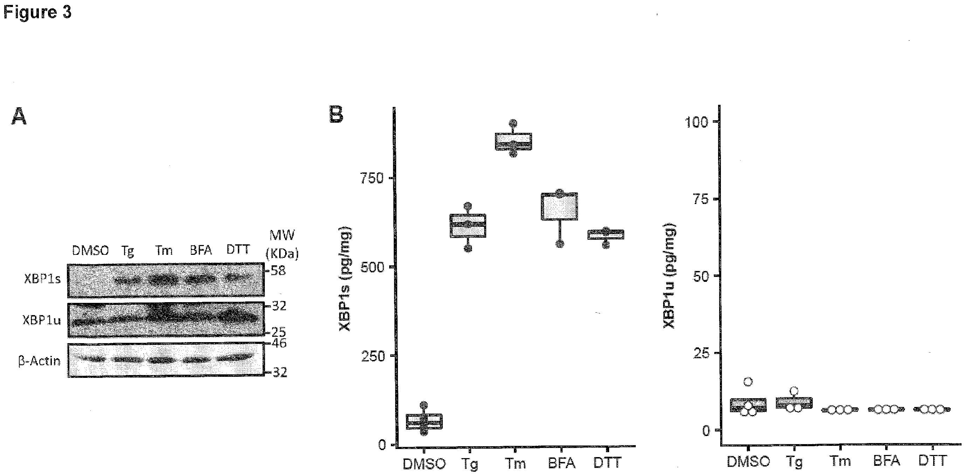

FIGURE 3 Quantified XBP1s upregulation upon pharmacologically induced ER stress. MDA-MB-231 cells were treated with UPR inducers Tg (0.5 mM, 48 h), Tm (1 µg/ml, 16 h), BFA (0.5 µg/ml, 16 h) and DTT (4 mM, 2 h) before being assessed by immunoblot (A) and XBP1 biochip (B).

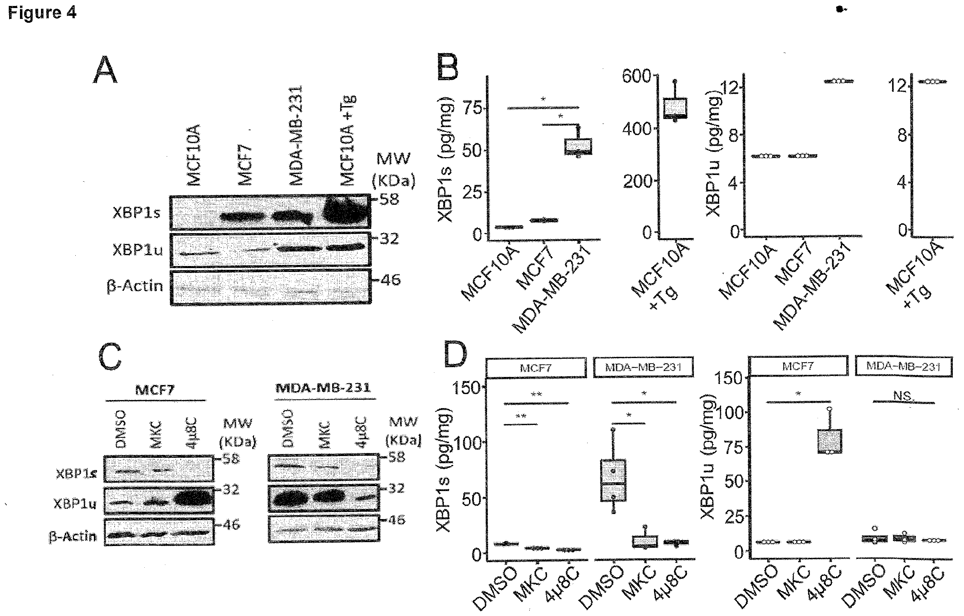

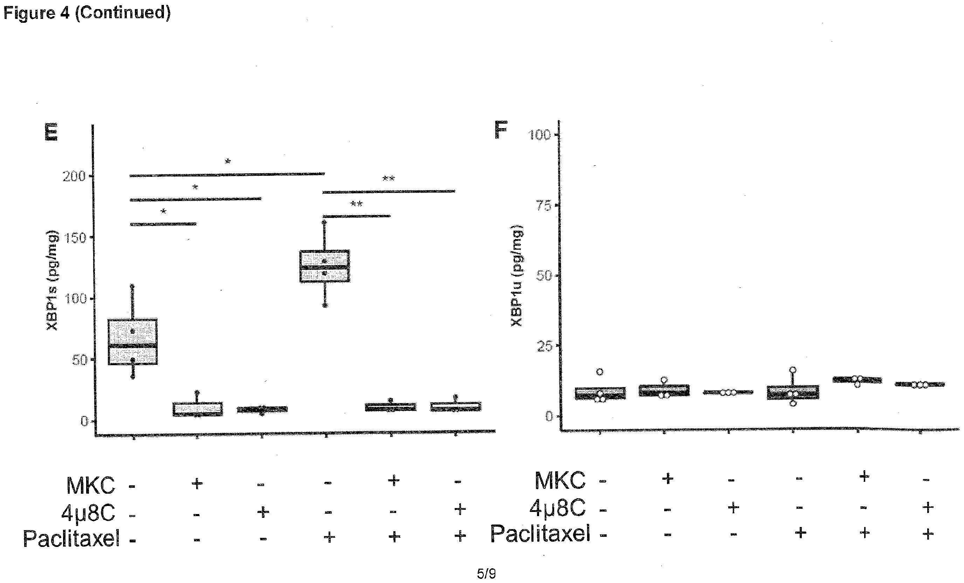

FIGURE 4 Basal and treatment dependent variation in XBP1 levels were quantified using the XBP1 biochip. XBP1s and XBP1u levels in MCF7, SKBR3 and MDA-MB-231 cell lines were assessed by immunoblot (A) and XBP1 biochip (B). MCF10A (± 0.5 mM Tg) were included as negative and positive controls. * indicates p<0.05. MCF7 and MDA-MB-231 cells were treated with vehicle (DMSO) or IRE1α RNase inhibitors 4µ8C (32 µM) or MKC-8866 (20 µM) for 48 h and assessed by immunoblot (C) and XBP1 biochip (D). XBP1 biochip assessment of MDA-MB-231 cells following 48 h treatment with XBP1 splicing inducing chemotherapeutic Paclitaxel (10 nM) and IRE1α RNase inhibition (E).

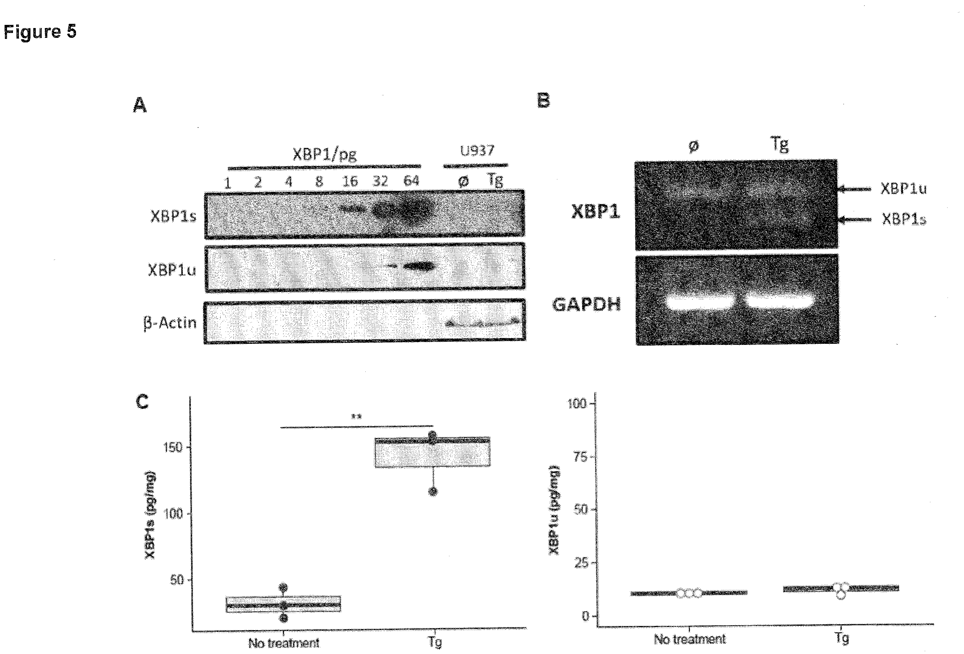

FIGURE 5 BAT technology detection of XBP1s and XBP1u levels in U937 lysates. 2.5×10⁶ untreated and Tg (0.25 mM, 48 h) treated U937 cells were lysed in RIPA and immunoblotted for XBP1s and XBP1u alongside a serial dilution of His-tagged recombinant protein. β-Actin acts as a loading control (A). RNA was extracted from the same cells and analysed by RT-PCR for XBP1 spicing. GAPDH serves as a control (B). RIPA lysed U937 cells were treated as in A and assessed by XBP1 biochip. ** p<0.01 (C).

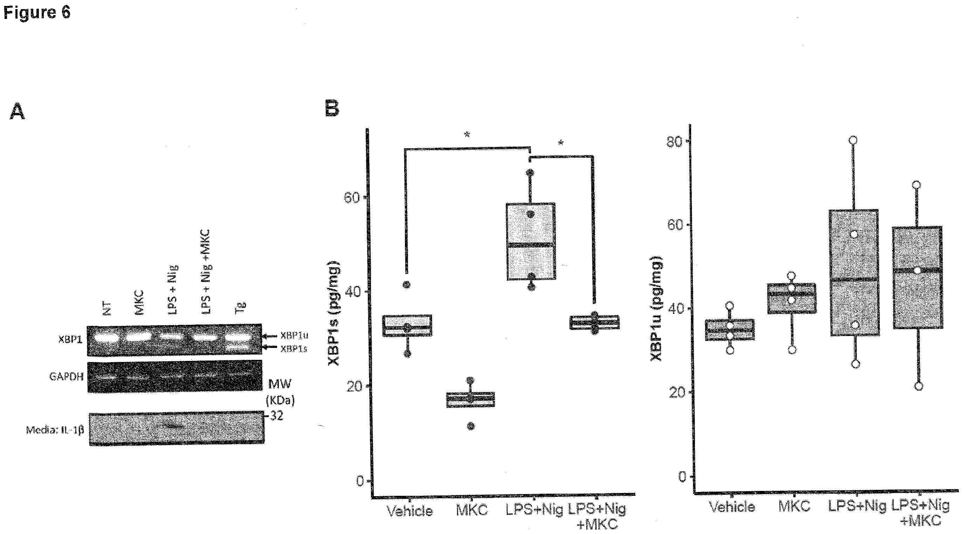

FIGURE 6 Multiplexed XBP1 assessment following inflammasome activation. THP-1 cells treated with LPS (1 µg/ml, 4 h) followed by nigericin (10 µM, 45 min), resulting in NLRP3 inflammasome activation and an increase in XBP1 splicing. Immunoblot of IL-1β in the conditioned media confirmed its release and inflammasome activation (A). XBP1 transcript and protein levels were assessed by RT-PCR (A) and biochip (B) respectively. * p<0.05.

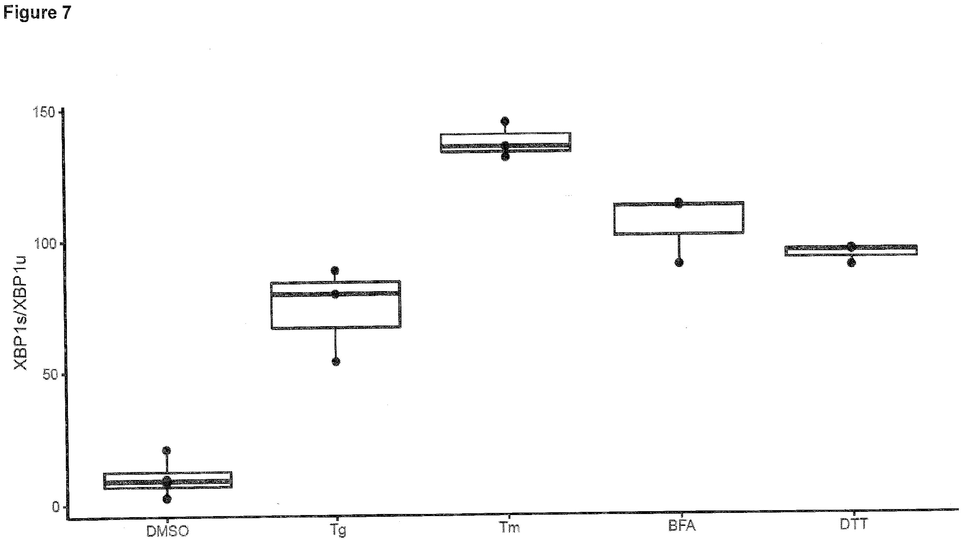

FIGURE 7 XBP1 protein ratio upon pharmacologically induced ER stress. XBP1 levels in MDA-MB-231 cells, expressed as a ratio of XBP1s (pg/mg)/XBP1u (pg/mg), upon induction with Tg (0.5 mM, 48 h), Tm (1 µg/ml, 16 h), BFA (0.5 µg/ml, 16 h) and DTT (4 mM, 2 h).

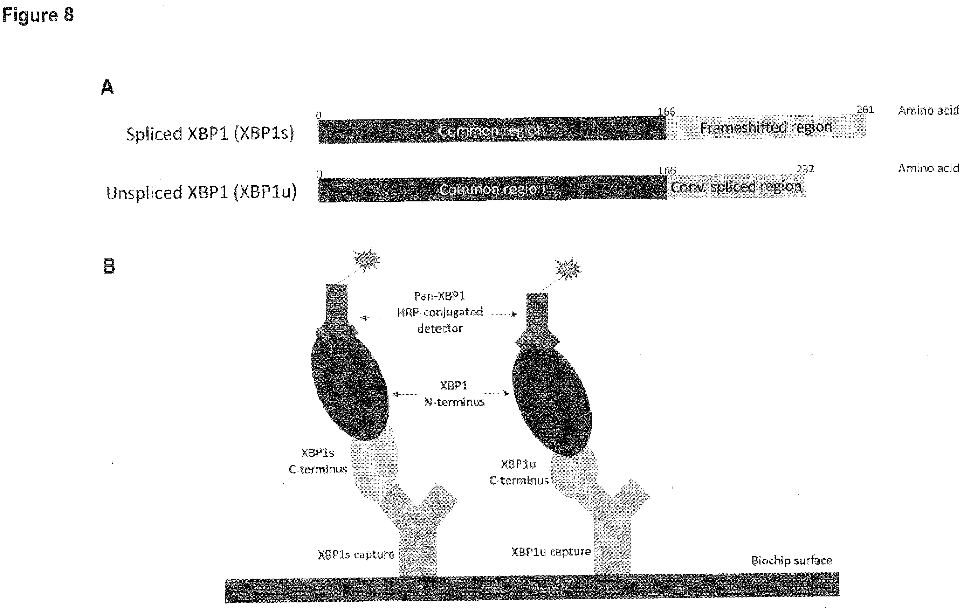

FIGURE 8 A sandwich immunoassay that captures the conventionally (conv.) spliced or frameshifted C-terminus of the XBP1 isoforms and a pan-detector of the common N-terminus was designed. Due to the unconventional splicing of XBP1 mRNA a translational frameshift occurs and results in XBP1 isoforms of differing length and C-terminal sequences (A). The biochip assay utilises these different C-termini for simultaneous capture and the common N-terminus for detection (B).

### DETAILED DESCRIPTION OF THE INVENTION

The invention described relates to products and processes for the detection or determination of the proteins XBP1u and XBP1s and their utility in the healthcare field for identifying processes and events relating to endoplasmic reticulum stress and the ascertainment of the unfolded protein response status, such utility including monitoring drug efficacy, confirming protein level changes in expression in relevant models and stratifying patients for targeted therapies.

A first aspect of the invention relates to a method of detecting or determining the proteins XBP1u and XBP1s comprising bringing an *in vitro* cell line or an *in vitro* biological sample taken from an individual into contact with a solid-state device supporting two antibodies at discrete locations, one antibody specific to XBP1u the second antibody specific to XBP1s; adding a further detector antibody, and determining the amount of each of XBP1s and XBP1u by comparison with a calibration curve, detecting the presence of XBP1s or XBP1u or determining the ratio of XBP1u and XBP1s.

In a preferred embodiment, the two antibodies are simultaneously exposed to the *in vitro* cell line or *in vitro* biological sample and the two subsequently bound or captured XBP1 isoforms detected or determined simultaneously. The simultaneous aspects of the assay method make for a more practical, error-free and efficacious process.

By 'detecting' is meant qualitatively analysing for the presence or absence of a substance e.g. a signal, usually above a set threshold value to account for background signal noise, indicating presence or absence of the substance; by 'determining' is meant quantitatively analysing for the amount of substance present. The individual can be healthy or diseased. The biological sample includes blood plasma, blood serum, urine, solid tumour extracts, peripheral blood mononuclear cells, bone marrow mononuclear cells, cerebrospinal fluid, cystic fluid or any suitable cell lysate. Following determination of the amount of each of XBP1s and XBP1u a ratio can be derived; alternatively, and advantageously, the ratio can be derived by a signal comparison from each XBP1 protein isoform, without the need to establish calibration curves using several known amounts (usually between five or nine different amounts) of the individual XBP1 isoforms. For example, the number of relative light units (RLUs) derived from the detectable output from each protein isoform can be measured and computed into a ratio, optionally by reference to a control value commonly used in immunoassays. The control value can be a single pre-determined threshold value and is derived from any suitable control e,g. a predetermined amount of XBP1s or XBP1u. The term 'specific' means that the antibody binds only to the relevant XBP1 isoform, with negligible binding to other isoform or to other analytes in the biological sample or cell line being analysed; this ensures that the integrity of the assay and the results derived are not compromised by additional binding events. The solid-state device can be a biochip, a microtitre plate, a nanoparticle, a slide or a bead system. A solid-state device may also be referred to as a substrate. Examples of bead materials are a single solid element such as carbon, silicon, silver, gold etc., an alloy, a mineral or a polymer or a combination of two or more materials. The antibodies engage with the substrate (i.e. supported by the substrate) by, for example, passive adsorption or can be chemically bonded to the substrate attached by way of, for example, covalent bonds. Such covalent bonding generally requires the initial introduction of a chemically active compound covalently attached to the substrate surface prior to antibody addition, leading to the so-called chemically-activated surface. The antibody itself may also require the addition of a chemical activating group to achieve substrate bonding. The attachment of the antibodies to the chemically-activated surface requires that the binding characteristics of the antibodies are not affected, and that the specificity and affinity of the two antibodies following the bonding process remain fit for purpose. Possible deleterious effects upon antibodies upon chemical activation and or bonding to the substrate include antibody include tertiary structure disruption leading to reduced bonding and/or specificity to the target epitope and compromised antibody orientation undermining contact between the antibody paratope and the target epitope; the current invention advantageously avoids both these possible effects. The substrate is preferably of a planar conformation such as a glass slide, microtitre plate or a biochip. A biochip is the preferred substrate due to its stability and adaptability. A biochip is a thin, wafer-like substrate with a planar surface which can be made of any suitable material such as glass or plastic but is preferably made of ceramic. The biochip is able to be chemically-activated prior to antibody bonding or is amenable to the passive adsorption of antibodies. Various aspects of biochip technology are described in EP0874242. A microlayer coating of material such as an ink composition can optionally be added to the planar surface of the substrate, preferably the biochip, prior to antibody placement, to increase the hydrophobic properties of the substrate and decrease non-specific binding of proteins in the biological sample or cell line. Either the upper surface or both surfaces of the substrate can be coated. The biochip can be integrated with or placed into a device with walls. Such a walled device can aid in the retention of added sample or solution. The solid-state device can also support other antibodies which have a binding specificity which is different from the binding specificity of the two XBP1 isoform antibodies. A support with multiple different antibodies is often described as a multianalyte array or multiplexing (array). Reference to an 'array' includes a microarray, the microarray characterised by the relatively small quantity of antibodies and the number of antibodies of different specificity placed onto the substrate at different locations. 'At discrete locations' implies, for a solid-state device such as a biochip which is a single surface devoid of barriers/partitions, that the individual specific antibodies are located at separate locations on the solid-state device surface; for a microtitre plate, which consists of multiple wells, the individual specific antibodies being located in separate wells. If a bead system is used then each specific antibody would normally be located on an individual bead.

The assay described in the invention is the sandwich immunoassay which proceeds by way of addition of the sample to be analysed followed by the binding of the analytes in the sample to antibodies (capture antibodies) adsorbed or bonded to the substrate surface, followed by addition of the detector antibody. The further antibody, the detector antibody, comprises a secondary antibody which binds to the XBP1 isoforms and promotes or provides a detectable and measurable signal enabling the captured XBP1 isoforms to be detected or quantified. The secondary antibody is preferably bound to an enzyme, bioluminescent or radioactive compound which are commonly used detectable labels; in a preferred embodiment of the methods and products of the invention the detector antibody comprises a secondary antibody conjugated to a detectable label that is an enzyme. Alternatively, the detector antibody comprises a first antibody derived from a species different from the capture antibody which can bind to each of the XBP1 isoforms, and a second antibody conjugated to a detectable label which binds specifically to the first antibody, wherein there is no overlapping cross-reactivity between the antibodies of the assay. The signal can be any suitable electromagnetic radiation based on for example phosphorescence, fluorescence, chemiluminescence (e.g. HRP/luminol system) etc. Preferably, fluorescence or chemiluminescence is used. An example of a detector antibody is a secondary antibody conjugated to biotin (Ab-biotin) which subsequently binds to a streptavidin-biotin-enzyme complex to give Ab-biotin-streptavidin-biotin-enzyme; a further example is a secondary antibody conjugated to biotin (Ab-biotin) which subsequently binds to a streptavidin-enzyme complex to give Ab-biotin-streptavidin-enzyme. Avidin can be used in place of streptavidin. A particularly preferred detector antibody is a secondary antibody bound to the detectable label horseradish peroxidase (HRP) i.e. Ab-HRP. A calibrator or standard, which can be used for effecting assay calibration, is well known in the art and enables a threshold concentration or the exact or calibrator equivalent amount of analyte(s) to be determined. The determination of an exact or calibrator equivalent amount of analyte(s) usually requires the construction of a calibration curve (also known as a standard curve). The number of calibrator points can vary, but is usually from 5 to 9. In the methods and products of the invention.

It is preferable that the antibody specific to XBP1u binds to an epitope incorporated within sequence 3 of XBP1u and that the antibody specific to XBP1s binds to an epitope incorporated within sequence 4 of XBP1s (see Figure 1). The two antibodies or capture antibodies, by binding to epitopes incorporated in sequences unique to XBP1u and XBP1s supports the specificity requirements of the method and minimises the possibility of each of the two capture antibodies binding to both XBP1 isoforms. For detection, one or two detector antibodies may be used. A single detector antibody binds to an epitope common to (present in) XBP1u and XBP1s which likely resides in Sequence 5 (Figure 1); alternatively, two detector antibodies one specific to an XBP1u epitope and one specific to an XBP1s epitope, can be used. It is preferable for ease and cost associated with assay development and production that a single detector antibody is used, preferably to an epitope incorporated in Sequence 5. It is preferable that the capture antibodies are bonded to a solid-state device, preferably a biochip, microtitre plate or beads. The biochip is particularly preferred due to its ease of handling during the development process and its preferred ceramic form, a mineral which is robust and has benign environmental properties. Furthermore, the biochip enables a sandwich immunoassay which is rapid, simplistic and which enables the simultaneous exposure of the two XBP1 isoform-specific capture antibodies to added sample and the simultaneous detection or quantification the two XBP1 isoforms, reducing the number of assay steps, reducing the intrinsic error and thus making the assay more accurate.

The invention further describes a solid state-device which supports two antibodies at discrete locations, one antibody specific to XBP1u the second antibody specific to XBP1s. Preferably, the solid-state device has a chemically reactive surface to which are bonded the two antibodies, one antibody specific to XBP1u the second antibody specific to XBP1s. In a preferred embodiment, the antibody specific to XBP1u binds to an epitope incorporated within sequence 3 of XBP1u and the antibody specific to XBP1s binding to an epitope incorporated within sequence 4 of XBP1s (see Figure 1 for all sequences referred to herein). The solid- state device can be incorporated in a kit. The kit may further incorporate a detector antibody which is preferably conjugated to a detectable label, the antibody specific to an epitope present within sequence 5. As stated previously, the detectable label may be any suitable detectable label chosen from an enzyme, bioluminescent compound or a radioactive compound, but is preferably horseradish peroxidase. The facility to detect or quantify XBP1s, XBP1u or both simultaneously, depending upon their presence in the sample being analysed, expands the utility of the assay and a solid-state device incorporating two capture antibodies of the requisite specificity.

To assess the XBP1 biochip's suitability and performance as a sandwich immunoassay in a multiplexed format the specificity and sensitivity of each assay was assessed. Two isoform specific capture antibodies, one to XBP1u and one to XBP1s, were spotted onto distinct discrete test regions (DTRs) on a ceramic biochip surface. Of the 25 DTRs present on a conventional biochip three are used for internal quality control by the analysis software (DTRs 4, 5 and 23). DTRs 8 and 14 were chosen for XBP1u (Figure 2A) and XBP1s analysis (Figure 2B) respectively. Clear signal with minimal background and cross-reactivity was observed upon spiking recombinant XBP1u (Figure 2A) or XBP1s (Figure 2B) into XBP1 assay calibrator base and assessing signal at the corresponding and other analyte's DTR. This DTR specificity and acceptable background signal (≤100 relative light units (RLU)) was reported even at 2.5 ng for both XBP1u (Figure 2C) and XBP1s (Figure 2D). When spiked with 6.5 ng of XBP1u or XBP1s cross reactivity at the other DTR was determined to be 0.61% and 0.30% respectively. It was possible to obtain a reliable, antigen dependent signal with consistent and reproducible RLU observed at a 0-650 pg calibration range (Figures 2E, F). The analytical sensitivity of each analyte was determined across 27 replicates and found to be 4.13 pg and 3.40 pg of XBP1u and XBP1u respectively while the functional sensitivity of each assay is 5.00 and 9.70 pg respectively. Multiplexed intra-assay precision was determined at two levels (-15-20 pg and -55-100 pg) for each analyte and remained below 10% for XBP1s and 15% for XBP1u across 27 replicates. These results demonstrate that the XBP1 biochip can reliably quantify the XBP1 isoforms with high specificity.

To confirm utility of the XBP1 biochip for cell lysate analysis a common model used to investigate ER stress mechanisms was utilised. Pharmacological induction of XBP1 splicing in *in vitro* and *in vivo* model systems is normally performed using a number of drugs with different mechanisms of action, all of which lead to an accumulation of misfolded proteins in the ER or a system unable to perform appropriate folding quality control. When MDA-MB-231 triple negative breast cancer (TNBC) cells were treated with Thapsigargin (Tg), Tunicamycin (Tm), Brefeldin A (BFA) or Dithiothreitol (DTT) an upregulation in XBP1s translation was observed (Figure 3i A). The XBP1 biochip was able to quantify the changes in XBP1 isoform expression, detecting the resultant XBP1 splicing at the protein level in RIPA lysates (Figure 3i B). A 10-30-fold increase in XBP1s/XBP1u ratio was detected by the XBP1 biochip when cells were treated with these commonly used treatments (Figure 3ii). This confirms the XBP1 biochip is also suitable for detection and analysis of pharmacologically induced XBP1 splicing using treatments commonly utilised in investigating the effects of UPR activation.

In order to test the XBP1 biochip in a physiologically relevant model basal XBP1 levels were assessed in several breast cancer cell lines of different subtypes. Recent studies have shown constitutive activation of IRE1α and resultant *XBP1* splicing in basal-like (when stratified molecularly) and triple negative (when stratified by receptor expression) breast cancers. Other breast cancer subtypes also display basal IRE1α activity. Immunoblots confirmed previous results in XBP1 isoforms when comparing non-tumorigenic, Oestrogen receptor positive (ER+) and triple negative breast cancer cell lines (MCF10A, MCF7 and MDA-MB-231 respectively) (Figure 4A). Using the XBP1 biochip we were able to observe and quantify these differences in XBP1 splicing at the protein level. TNBC cell line MDA-MB-231 displayed significantly higher basal XBP1 splicing than the non-tumorigenic or ER+ cell lines (p= 0.014 and 0.012 respectively). MCF7 cells also show low level IRE1α activity, with a mean XBP1s expression of 7.78pg/mg (S.E.= 0.278pg/mg). Reported XBP1u levels were consistently within the functional sensitivity of the assay of 9.70 pg, indicating low basal expression levels (Figure 4B).

To test if the XBP1 biochip has suitable sensitivity for research applications IRE1α RNase activity was pharmacologically inhibited. Inhibition of basal IRE1α RNase activity has previously been shown to reduce tumour progression and size *in vitro* and *in vivo* in murine models. Immunoblot was used to confirm a reduction in XBP1s both in higher expressing MDA-MB-231 cells and lower expressing MCF7 cells when using IRE1α RNase inhibiting compounds 4µ8C and MKC-8866 (Figure 4C). When quantified by XBP1 biochip, 4µ8C mediated inhibition appeared to be more potent than MKC but use of both compounds resulted in significant reductions in XBP1s in MCF7 (p=0.004 and p=0.008) and MDA-MB-231 models (p=0.035 and p=0.033). XBP1u protein levels remained largely unchanged in the MDA-MB-231 model during IRE1α inhibition (p=0.71). However, MCF7 cells showed a significant upregulation of XBP1u protein upon 4µ8C treatment (p=0.019) with expression levels averaging 81.1 pg/mg (Figure 4D).

To assess the biochip's applicability in pre-clinical models a currently clinically approved modulator of IRE1α activity was used. Paclitaxel, a commonly used chemotherapeutic in TNBC treatment, has also been shown to induce XBP1 splicing. Here we demonstrated that this increase in XBP1s is observed at the protein level and Paclitaxel induced and pharmacologically inhibited XBP1s regulation is quantifiable with the XBP1 biochip. Paclitaxel treatment of MDA-MB-231 cells resulted in a significant increase in XBP1s expression (66.9±16.2 pg/mg to 125.0±14.1 pg/mg, p=0.036). This increase was completely ablated by pharmacological inhibition of IRE1α RNase activity (p<0.01 upon MKC-8866 or4µ8C treatment). XBP1u levels showed no significant change with Paclitaxel, MKC-8866 and/or 4µ8C treatment (Figure 4E). These data indicate the utility of the XBP1 biochip in relevant pre-clinical models, showing its ability to detect basal and treatment induced levels of the XBP1 isoforms. Quantification of treatment-induced changes resulted in significant differences in XBP1s levels of cells treated with IRE1α inhibitors MKC-8866 and 4µ8C, and clinically relevant ER stress inducer Paclitaxel.

To determine if the XBP1 biochip had advantages over immunoblotting (the current standard method of protein level detection) a non-adherent cell model was assessed. Detecting the XBP1 isoforms by immunoblotting can be difficult, with many researchers preferring to detect *XBP1* splicing at the mRNA level. Assessment of proteins of low abundance by immunoblotting can be particularly difficult in non-adherent cells. This difficulty is typified in U937 cells, where even after achieving exceptionally high sensitivity (low picogram levels) it was still not possible to detect either XBP1 isoform in unstimulated or Tg stimulated samples by immunoblot even with high protein input and prolonged exposure of the x-ray film (Figure 5A). Detection difficulty was not due to lack of *XBP1* expression or IRE1α RNase activity as both presence and splicing of the transcript was confirmed using RT-PCR (Figure 5B). In contrast, the XBP1 biochip was able to detect significant changes in XBP1s protein expression in the same samples (p=0.0056, Figure 5C).This demonstrates that the XBP1 biochip can be used to assess both XBP1 isoforms' protein levels even in model systems where XBP1 immunoblots can be difficult. The immunoblots shown here took approximately 72 h whilst total assessment using the XBP1 biochip took ~3 h.

To confirm the applicability of the XBP1 biochip in a relevant non-adherent model system pro-monocytic THP-1 cells were assessed upon inflammatory release. It has recently been demonstrated that XBP1s splicing occurs upon activation the NLRP3 inflammasome and that inhibition of IRE1α RNase activity could ablate NLRP3 mediated IL-1β release. It is demonstrated that this XBP1s splicing is detectable after only 4 h of LPS priming and 45 min of Nigericin secondary signal treatment and we replicate the previous observation that MKC-8866 mediated inhibition of IRE1α RNase activity ablates IL-1β release (Figure 6A). The XBP1 biochip was used to detect and quantify these effects on XBP1 splicing at the protein level, detecting significant changes in XBP1s expression upon inflammasome activation (p=0.044) and MKC-8866 mediated inhibition (p=0.048). XBP1u levels remain relatively high (mean= 46.2 pg/mg) but are largely unchanged by treatment (one-way ANOVA, p=0.408) (Figure 6B). Thus, the XBP1 biochip can detect XBP1 splicing following inflammatory stimulation and has identified a previously unreported high level of XBP1u protein in THP-1 cells. The comparison of the detection and determination measurements for XBP1u and XBP1s in the ER unstressed and stressed states thus allows for the categorisation of cell lines and patient samples and has particular utility in assessing drug efficacy in various disease states.

Thus a further aspect of the invention is a method of detecting or determining the proteins XBP1u and XBP1s comprising bringing an *in vitro* cell line or an *in vitro* biological sample taken from an individual into contact with a solid-state device supporting two antibodies at discrete locations on the solid-state device, one antibody specific to XBP1u the second antibody specific to XBP1s; adding at least one further detector antibody, and determining the amount of each of XBP1u and XBP1s by comparison with a calibration curve, detecting the presence of XBP1s or XBP1u and based upon the detection or determination measurements ascertaining the unfolded protein response status of the cell line or biological sample taken from the individual; preferably, the detection or determination of the two protein isoforms takes place simultaneously. In an embodiment, the ratio of XBP1u and XBP1s is measured, making the requirement for a calibration curve optional and decreasing the likelihood of miscalculation and erroneous measurement results that can be introduced when cell line and biological sample dilutions take place. Production of or an increase of XBP1s signifies the presence of stress in the endoplasmic reticulum and an increase in the unfolded protein response as does an increase in the XBP1s to XBP1u ratio. The biological sample can be blood plasma, blood serum, urine, solid tumour extracts, peripheral blood mononuclear cells, bone marrow mononuclear cells, cerebrospinal fluid, cystic fluid or any suitable cell lysate. An indication of endoplasmic reticulum stress and that an unfolded protein response in a cell line or a biological sample taken from an individual exists or has increased or decreased following exposure to a drug, can be derived by measuring the level of XBP1s, XBP1u or their ratio before drug exposure and comparing this measurement to a measurement of XBP1s, XBP1u or their ratio after drug exposure; alternatively, the indication can be derived by measuring the level of XBP1s, XBP1u or their ratio in the cell line or the biological sample taken from an individual and comparing the measurement(s) to a control value, the control value being a stored database value of XBP1s, XBP1u or their ratio values of known unfolded response status, or XBP1s and/or XBP1u measurements derived from the individual's biological sample at an earlier time point.

A rapid, reliable and quantitative method of detecting both XBP1 isoforms at the protein level in several relevant model systems is exemplified. Importantly, the XBP1 biochip has also demonstrated the divergence of protein levels of XBP1s and XBP1u from their transcript levels. XBP1s isoform transcript levels show correlation with XBP1s isoform protein levels, whereas XBP1u isoform levels for transcript and protein do not correlate. In all but the most severe responses to ER stress XBP1u is the dominant transcript but at the protein level this pattern is reversed and there are lower levels of XBP1u relative to XBP1s, particularly under ER stress (van Schadewijk A et al). XBP1s/XBP1u mRNA ratios are commonly used as a measure of IRE1α activity in the literature (Logue SE, McGrath EP et al) which can lead to erroneous interpretations and the finding that XBRP1s to XBPR1u protein ratios is a more accurate reflection, is a clinically beneficial finding. The IRE1α mediated unconventional splicing of XBP1 pre-mRNA, resulting in the differential expression of the XBP1 isoforms, is a biomarker and a druggable target in various disease states; the method and solid-state device for XBP1s and XBP1u isoform detection and quantification reported herein enables their efficient and beneficial exploitation and can be used for basal protein expression, disease prognostics and drug efficacy prediction by assessing the levels and ratio of XBP1s and XBP1u in cell lines and biological samples before and after drug exposure.

### Material and Methods

The term XBP1 or'X-box binding protein 1' refers to Uniprot number P17861; Isoform 1 is XBP1u with Uniprot number P17861-1 and sequence 1 of Figure 1; Isoform 2 is XBP1s with Uniprot number P17861-2 and sequence 2 of Figure 1. *Reagents* XBP1 Biochip assay kit was obtained from Randox Laboratories, Randox Laboratories Ltd., Crumlin, UK) comprising two capture antibodies one specific to XBP1u and one specific to XBP1s discretely located on a biochip and a detector antibody specific to a epitope common to XBP1u and XBP1s. Thapsigargin (Tg) (Sigma-Aldrich, St. Louis, USA, St. Louis, USA), Tunicamycin (Tm) (Sigma-Aldrich, Sigma-Aldrich, St. Louis, USA), Brefeldin A (BFA) (Sigma-Aldrich, St. Louis, USA), Dithiothreitol (DTT) (Sigma-Aldrich, St. Louis, USA), 4µ8C (Sigma-Aldrich, St. Louis, USA), MKC-8866 (Probechem, St Pete Beach, USA), Paclitaxel (Taxol) (Sigma-Aldrich, St. Louis, USA), Lipopolysaccharide (LPS) (Sigma-Aldrich, St. Louis, USA) and Nigericin (Nig) (Invivogen, San Diego, USA) were diluted in DMSO (Sigma-Aldrich, St. Louis, USA).

*Biochip-based determination* of *XBP1 and XBP1u.* XBP1s and XBP1u levels and raw signal were quantified using the Evidence Investigator analyser (EV3602, Randox Laboratories Ltd., UK). Signals from defined discrete test regions were detected using digital imaging technology. The biochips were provided in wells in a carrier in a 3x3 format (9 reaction wells per carrier). A carrier handling tray supplied with the system accommodated 6 carriers. The total assay protocol was performed according to manufacturer's instructions. Briefly, RIPA lysed samples were diluted to 100 µL in RIPA buffer followed by dilution to 200 µL in XBP1 assay buffer and mixed well with gentle pipetting. 200 µL of XBP1 assay diluent was then applied to the surface of the biochip followed by 200 µL diluted sample or 100 µL calibrator/control per well. Following a 60-minute incubation at 37 °C, 370 RPM in a thermoshaker (Randox Laboratories Ltd., Crumlin, UK) liquid contents were removed with a sharp flick and washed in a 2X quick, 4X 2 min wash steps. 300 µL of HRP conjugated pan-XBP1 detector was applied to each well and another 60 min incubation at 37 °C, 370 RPM in a thermoshaker was performed. Following another wash as above 250 µL of a 1:1 ratio of EV841 luminol and peroxide was applied to the surface of each biochip in a carrier and incubated away from direct light for 2 min. Carriers were then submitted to the Evidence Investigator, light emitted from each DTR detected by the CCD camera and signal quantified by the instrument software. Final values in pg/mg were obtained by dividing reported value (in pg) by total protein loaded per well, as determined by bicinchoninic acid (BCA) assay. Calibration curves, inferred values and goodness of fit were independently confirmed using R package nplr (0.1-7, Frederic Commo, https://cran.r-project.org/web/ packages/nplr/index.html).

*Cell culture and treatments* MCF10A (ATCC) cells were maintained in DMEM/F-12 (Sigma-Aldrich, St. Louis, USA) supplemented with 5% horse serum (Sigma-Aldrich, St. Louis, USA), 20 ng/mL epidermal growth factor (PeproTech, London, UK), 0.5 µg/mL Hydrocortisone (Sigma-Aldrich, St. Louis, USA), 100 ng/mL Cholera toxin (Sigma- Aldrich, St. Louis, USA), 10 µg/mL insulin (Sigma-Aldrich, St. Louis, USA), 50 U/mL¹ penicillin, and 50 µg/mL streptomycin (St. Sigma-Aldrich, St. Louis, USA). MCF7 cells (ECACC) were cultured in DMEM high glucose (Sigma-Aldrich, St. Louis, USA) supplemented with 10% FBS, 0.01 mg/mL Insulin (Sigma-Aldrich, St. Louis, USA), and 2 mM L-glutamine. MDA-MB-231 cells were cultured in DMEM high glucose (Sigma-Aldrich, St. Louis, USA) supplemented with 10% FBS, 50 U/mL' penicillin, 50 µg/mL streptomycin, and 2 mM L-glutamine. U937 and THP-1 (ATCC, Manassas, USA) cells were cultured in RPMI-1640 medium (Sigma-Aldrich, St. Louis, USA) supplemented with 10% foetal bovine serum (FBS) (Sigma-Aldrich, St. Louis, USA), 50 U/mL penicillin, 50 µgml⁻¹ streptomycin (Sigma-Aldrich, St. Louis, USA), and 2mM L-glutamine (Sigma-Aldrich, St. Louis, USA). All cells were cultured at 37 °C, 5% CO₂ in a humidified incubator and adherent cells seeded at an appropriate density 24 h prior to treatment. U937 cells were seeded at 5×10⁵ cells/mL and treated immediately while THP-1 cells were seeded at 1×10⁶ cells/mL and treated after 2 hours.

*Sample preparation* Cells were washed once in ice cold phosphate buffered saline (PBS) and then lysed in either RIPA buffer (Sigma-Aldrich, St. Louis, USAUSA) with RocheSTOP protease inhibitors (Roche, Basel, Switzerland) for protein analysis or directly lysed in TriReagent (Sigma-Aldrich, St. Louis, USAUSA) as per manufactures instructions for RNA analysis.

*Immunoblotting* Protein lysates were mixed with 5X Laemmli Buffer (0.3125 M Tris HCl (pH 6.8), 10% SDS, 50% glycerol, 25% 2-mercaptoethanol, 0.02% bromophenol blue) in a 1:4 ratio and boiled at 95 °C for 5 min. Samples were separated on an SDS polyacrylamide gel, transferred onto nitrocellulose membrane (GE Healthcare Life Sciences, Little Chalfont, UK) and blocked with 5% milk in Wash Buffer (Randox Laboratories Ltd., Crumlin, UK). Membranes were probed with commercial antibodies to spliced and unspliced XBP1 isoforms and Actin (Sigma-Aldrich, St. Louis, USA, 1:5000). Anti-rabbit (Jackson ImmunoResearch, Cambridge, UK) and anti-mouse (Jackson ImmunoResearch, Cambridge, UK; Sigma-Aldrich, St. Louis, USA) HRP-conjugated secondary antibodies were incubated for 45-60 min and the signal was visualized using western blotting luminol reagent (Thermofisher, Waltham, USA; Perkin-Elmer, Waltham, USA).

*RT-PCR* 500-5000 ng of purified RNA was reverse transcribed using Superscript II (Thermofisher, Waltham, USA). PCR was performed using GoTaq Green (MyBio Ltd, Kilkenny, Ireland) master mix and the following primers: FW XBP1 *5'-GGA ACA*

*GCA AGT GGT AGA-3',* RVXBP1 *5'-CTG GAG GGG TGA CAA CTG-3',* FW GAPDH 5'-ACC ACA GTC CAT GCC ATC-3' and RV GAPDH *5'-TCC ACC ACC*

*CTG TTG CTG-3'.* Products were visualised on 3-4% Agarose in Tris Base, Acetic Acid, EDTA (TAE) buffer gels and stained with Midori green (ANACHEM, Leicester, UK).

*Statistical analysis* Statistical analysis was carried out using two-tailed t test with Welch's correction or one-way ANOVA where appropriate. *P* < 0.05 was considered statistically significant. Final analysis and calculations were performed in R version 3.5.1 "Feather Spray".

### References

Logue SE, McGrath EP et al. (2018). Inhibition of IRE1 RNase activity modulates the tumor cell secretome and enhances response to chemotherapy. Nature Communications, 9:3267.

van Schadewijk A, van't Wout EFA, Stolk J, Hiemstra PS (2012). A quantitative method for detection of spliced X-box binding protein-1 (XBP1) mRNA as a measure of endoplasmic reticulum (ER) stress. Cell Stress Chaperones, 17(2): 275-279.

## Claims

1. A method of detecting or determining the proteins XBP1u and XBP1s comprising bringing an *in vitro* cell line or an *in vitro* biological sample taken from an individual into contact with a solid-state device supporting two antibodies at discrete locations on the solid-state device, one antibody specific to XBP1u the second antibody specific to XBP1s; adding at least one further detector antibody, and detecting or determining the amount of XBP1u and XBP1s.

2. The method of claim 1 in which the antibody specific to XBP1u binds to an epitope incorporated within sequence 3 of XBP1u as shown in Figure 1 and the antibody specific to XBP1s binds to an epitope incorporated within sequence 4 of XBP1s as shown in Figure 1.

3. The method of claims 1or 2 in which the two isoform-specific XBP1 antibodies are simultaneously exposed to the *in vitro* cell line or *in vitro* biological sample and the XBP1u and the XBP1s proteins are detected or determined simultaneously.

4. The method of any of the preceding claims in which the amount of XBP1u and XBP1s is determined by comparison with a calibrator curve or following their determination an XBP1s and XBP1u ratio is derived.

5. The method of any of the preceding claims in which the solid-state device has a chemically reactive surface to which the two antibodies are bonded.

6. The method of any of the preceding claims in which the detector antibody is specific to an epitope present in both XBP1u and XBP1s.

7. The method of any of the preceding claims in which the detector antibody is specific to an epitope present within sequence 5 of Figure 1.

8. The method of any of the preceding claims in which based upon the detection or determination of XBP1s and XBP1u the unfolded protein response status of the cell line or biological sample taken from the individual is ascertained.

9. The method of claim 8 in which the unfolded protein response status of the cell line or biological sample taken from the individual is categorised as increased if the XBP1s level or the XBP1s to XBP1u ratio is increased.

10. A solid-state device which supports two antibodies at discrete locations, one antibody specific to XBP1u the second antibody specific to XBP1s.

11. The solid-state device of claim 10 which has a chemically reactive surface to which are bonded the two antibodies, one antibody specific to XBP1u the second antibody specific to XBP1s.

12. The solid-state device of claim 10 or 11 in which the antibody specific to XBP1u binds to an epitope incorporated within sequence 3 of XBP1u as shown in Figure 1 and the antibody specific to XBP1s binds to an epitope incorporated within sequence 4 of XBP1s as shown in Figure 1.

13. A kit for the detection of XBP1u and XBP1s comprising the solid-state device of any of claims 11 to 12.

14. The kit of claim 13 further comprising a detector antibody specific to an epitope present within sequence 5 of Figure 1.

15. Use of the solid-state device or kit of claims 10 to 14 in a process for ascertaining the effect of a drug upon the unfolded protein response in an *in vitro* cell line or *in vitro biological* sample, preferablywherein during the process the ratio of XBP1s to XBP1u is derived.

## Patentansprüche

1. Verfahren zum Nachweis oder zur Bestimmung der Proteine XBP1u und XBP1s, umfassend Inkontaktbringen einer *In-vitro-*Zelllinie oder einer biologischen *In-Vitro*-Probe, die einer Person entnommen wurde, mit einer Festkörpervorrichtung, die zwei Antikörper an getrennten Stellen auf der Festkörpervorrichtung trägt, wobei ein Antikörper spezifisch für XBPlu, der zweite Antikörper spezifisch für XBP1s ist; wobei mindestens ein weiterer Detektorantikörper hinzugefügt wird und die Menge von XBP1u und XBP1s nachgewiesen oder bestimmt wird.

2. Verfahren nach Anspruch 1, bei dem der für XBP1u spezifische Antikörper an ein Epitop, das in die Sequenz 3 von XBP1u integriert ist, bindet, wie in Figure 1 gezeigt, und der für XBP1s spezifische Antikörper an ein Epitop, das in die Sequenz 4 von XBP1s integriert ist, bindet, wie in Figure 1 gezeigt.

3. Verfahren nach Anspruch 1 oder 2, bei dem die zwei isoformspezifischen XBP1-Antikörper gleichzeitig der *In-vitro*-Zelllinie oder biologischen *In-vitro*-Probe ausgesetzt werden und die XBP1u- und die XBP1s-Proteine gleichzeitig nachgewiesen oder bestimmt werden.

4. Verfahren nach einem der vorhergehenden Ansprüche, bei dem die Menge von XBP1u und XBP1s durch einen Vergleich mit einer Kalibrierkurve bestimmt wird oder nach ihrer Bestimmung ein XBP1s- und XBP1u-Verhältnis abgeleitet wird.

5. Verfahren nach einem der vorhergehenden Ansprüche, bei dem die Festkörpervorrichtung eine chemisch reaktive Oberfläche hat, an die die beiden Antikörper gebunden werden.

6. Verfahren nach einem der vorhergehenden Ansprüche, bei dem der Detektorantikörper für ein sowohl in XBP1u als auch in XBP1s vorhandenes Epitop spezifisch ist.

7. Verfahren nach einem der vorhergehenden Ansprüche, bei dem der Detektorantikörper für ein Epitop, das in Sequenz 5 von Figure 1 vorhanden ist, spezifisch ist.

8. Verfahren nach einem der vorhergehenden Ansprüche, bei dem basierend auf dem Nachweis oder der Bestimmung von XBP1s und XBP1u der ungefaltete Proteinantwortzustand der Zelllinie oder biologischen Probe, die von der Person entnommen wurde, ermittelt wird.

9. Verfahren nach Anspruch 8, bei dem der ungefaltete Proteinantwortzustand der Zelllinie oder biologischen Probe, die von der Person entnommen wurde, als erhöht eingestuft wird, falls der XBP1s-Wert oder das Verhältnis XBP1s zu XBP1u erhöht ist.

10. Festkörpervorrichtung, die zwei Antikörper an getrennten Stellen trägt, wobei ein Antikörper spezifisch für XBP1u, der zweite Antikörper spezifisch für XBP1s ist.

11. Festkörpervorrichtung nach Anspruch 10, die eine chemisch reaktive Oberfläche hat, an die die beiden Antikörper gebunden sind, wobei ein Antikörper spezifisch für XBP1u, der zweite Antikörper spezifisch für XBP1s ist.

12. Festkörpervorrichtung nach Anspruch 10 oder 11, bei der der für XBP1u spezifische Antikörper an ein Epitop, das in die Sequenz 3 von XBP1u integriert ist, bindet, wie in Figure 1 gezeigt, und der für XBP1s spezifische Antikörper an ein Epitop, das in die Sequenz 4 von XBP1s integriert ist, bindet, wie in Figure 1 gezeigt.

13. Set zum Nachweis von XBP1u und XBP1s, umfassend die Festkörpervorrichtung nach einem der Ansprüche 11 bis 12.

14. Set nach Anspruch 13, ferner umfassend einen Detektorantikörper, der für ein in Sequenz 5 von Figure 1 vorhandenes Epitop spezifisch ist.

15. Anwendung der Festkörpervorrichtung oder des Sets nach den Ansprüchen 10 bis 14 in einem Prozess zur Ermittlung der Wirkung eines Arzneimittels auf die ungefaltete Proteinantwort in einer *In*-*vitro*-Zelllinie oder einer biologischen *In-vitro*-Probe*,* wobei vorzugsweise während des Prozesses das Verhältnis von XBP1s zu XBP1u abgeleitet wird.

## Revendications

1. Procédé de détection ou de détermination des protéines XBP1u et XBP1s comprenant mettre une lignée cellulaire *in vitro* ou un échantillon biologique *in vitro* prélevé d'un individu en contact avec un dispositif à semi-conducteur portant deux anticorps à des emplacements discrets sur le dispositif à semi-conducteur, un anticorps spécifique à XBP1u, le deuxième anticorps spécifique à XBP1s ; ajouter au moins un anticorps détecteur en plus, et détecter ou déterminer la quantité de XBP1u et de XBP1s.

2. Procédé selon la revendication 1, dans lequel l'anticorps spécifique à XBP1u se lie à un épitope incorporé dans la séquence 3 de XBP1u comme illustré sur la Figure 1, et l'anticorps spécifique à XBP1s se lie à un épitope incorporé dans la séquence 4 de XBP1s comme illustré sur la Figure 1.

3. Procédé selon les revendications 1 ou 2, dans lequel les deux anticorps spécifiques à XBP1 isoformes sont exposés simultanément à la lignée cellulaire *in vitro* ou à l'échantillon biologique *in vitro,* et les protéines XBP1u et XBP1s sont détectées ou déterminées simultanément.

4. Procédé selon l'une quelconque des revendications précédentes, dans lequel la quantité de XBP1u et de XBP1s est déterminée par comparaison à une courbe étalon ou bien suite à la détermination un rapport de XBP1s et de XBP1u est dérivé.

5. Procédé selon l'une quelconque des revendications précédentes, dans lequel le dispositif à semi-conducteur a une surface chimiquement réactive à laquelle les deux anticorps sont liés.

6. Procédé selon l'une quelconque des revendications précédentes, dans lequel l'anticorps détecteur est spécifique à un épitope présent dans XBP1u et XBP1s toutes les deux.

7. Procédé selon l'une quelconque des revendications précédentes, dans lequel l'anticorps détecteur est spécifique à un épitope présent dans la séquence 5 de la Figure 1.

8. Procédé selon l'une quelconque des revendications précédentes, dans lequel sur la base de la détection ou de la détermination de XBP1s et de XBP1u, l'état de réponse de protéines dépliées de la lignée cellulaire ou de l'échantillon biologique prélevé de l'individué est vérifié.

9. Procédé selon la revendication 8, dans lequel l'état de réponse de protéines dépliées de la lignée cellulaire ou de l'échantillon biologique prélevé de l'individu est catégorisé comme augmenté si le taux de XBP1s ou le rapport de XBP1s à XBP1u est augmenté.

10. Dispositif à semi-conducteur qui porte deux anticorps à des emplacements discrets, un anticorps spécifique à XBP1u, le deuxième anticorps spécifique à XBP1s.

11. Dispositif à semi-conducteur selon la revendication 10, qui a une surface chimiquement réactive à laquelle les deux anticorps sont liés, un anticorps spécifique à XBP1u, le deuxième anticorps spécifique à XBP1s.

12. Dispositif à semi-conducteur selon la revendication 10 ou 11, dans lequel l'anticorps spécifique à XBP1u se lie à un épitope incorporé dans la séquence 3 de XBP1u comme illustré sur la Figure 1, et l'anticorps spécifique à XBP1s se lie à un épitope incorporé dans la séquence 4 de XBP1s comme illustré sur la Figure 1.

13. Kit de détection de XBP1u et de XBP1s comprenant un dispositif à semi-conducteur selon l'une quelconque des revendications 11 à 12.

14. Kit selon la revendication 13, comprenant en outre un anticorps détecteur spécifique à un épitope présent dans la séquence 5 de la Figure 1.

15. Utilisation du dispositif à semi-conducteur ou du kit selon les revendications 10 à 14, dans un procédé de vérification de l'effet d'un médicament sur la réponse de protéines dépliées dans une lignée cellulaire *in vitro* ou dans un échantillon biologique *in vitro,* de préférence où durant le procédé le rapport de XBP1s à XBP1u est dérivé.
